# EUROPEAN PATENT SPECIFICATION

(11) **EP 1 581 563 B1**
(45) Date of publication and mention of the grant of the patent: **05.07.2006**
(21) Application number: 03799589.1
(22) Date of filing: 18.12.2003
(51) Int. Cl.: C08F 2/32, A61K 8/04, A61K 8/81, A61Q 19/00

(54) **SYNTHETIC THICKENERS FOR COSMETICS**
SYNTHETISCHE VERDICKUNGSMITTEL FÜR KOSMETIKA
AGENTS EPAISSISSANTS SYNTHETIQUES POUR PRODUITS COSMETIQUES

(30) Priority: 09.01.2003 IT VA20030002
(43) Date of publication of application: 05.10.2005
(73) Proprietor: Lamberti Spa, 21041 Albizzate (IT)
(72) Inventor: POLOTTI, Gianmarco, I-20099 Sesto San Giovanni (IT); BENETTI, Arianna, I-21013 Gallarate (IT); FEDERICI, Franco, I-21052 Busto Arsizio (IT); LI BASSI, Giuseppe, I-21026 Gavirate (IT)
(86) International application number: PCT/EP2003/051056
(87) International publication number: WO 2004/063228

(56) References cited:
- EP-A- 0 172 723
- EP-A- 0 562 344
- US-B1- 6 197 287

## Description

### TECHNICAL FIELD

The present invention relates to inverse emulsions useful as thickeners in cosmetic formulations and to the procedure for their preparation.

Cosmetic formulations include all the products normally used for personal care, such as body and face creams, cleansing fluids, after-shave balms, foundation creams and other products for similar applications.

### BACKGROUND ART

It is known that a technical problem often encountered in the cosmetic industry is to obtain high viscous formulations (pastes, gels) which are stable over time.

An essential characteristic of the thickeners employed in cosmetic formulations is that they manifest their thickening capability even when used in small quantities, without negatively altering the other properties of the formulations.

In the specialised literature many methods are reported to regulate the rheological properties of different formulations, often including the use of polymers in the form of inverse emulsion (an inverse emulsion is an emulsion containing both an oil-in-water emulsifier and a water-in-oil emulsifier, wherein the aqueous phase is dispersed in the organic phase in very small drops).

We cite, as an example, EP 503853, wherein an inverse emulsion containing a polymer comprising units deriving from acrylamide, 2-acrylamido-2-methylpropanesulfonic acid and a polyfunctional monomer is described.

A disadvantage of the inverse emulsions of EP 503853 is the fact that they may contain traces of acrylamide monomer, a toxic substance which is unacceptable by the present European legislative trend.

In US 6,375,959 and US 6,197,287 a procedure for the preparation of cross-linked or branched anionic polyelectrolytes based on strongly acidic monomers and other monomers (but not acrylamide nor hydrophobic monomers), in the form of an inverse emulsion, is described.

The lack of stability of the emulsions used as thickeners in cosmetics, even if it is not a determining characteristic in view of the final properties of the finished cosmetic product itself, may cause troubles during their preparation, storing and transport.

It is highly desirable in the cosmetic field to have thickeners in the form of emulsion that, besides conferring a perfect homogeneity and showing both good thickening efficiency in different conditions and ease of use, are commercially available as stable emulsions and are able to give stable cosmetic formulations.

With the expression "stable emulsion" we mean an emulsion that in the normal storing conditions (from -10°C to 40°C) and for the usual lifetime (180-360 days) does not show phase separation, sediment, formation of floating pellicles and lumps.

With the expression "stable cosmetic formulation" we mean a thickened cosmetic formulation that in the above said conditions and lifetime does not show phase separation, sediment, formation of floating pellicles and lumps.

### DISCLOSURE OF INVENTION

It has now surprisingly been found that the inverse emulsions containing an anionic acrylic polymer obtained by inverse emulsion polymerisation of one or more anionic acrylic monomers, at least one of which containing a strongly acidic functional group, dissolved in the aqueous phase, and at least a hydrophobic acrylic monomer dissolved in the oil phase before the mixing of the two phases, possess a stability which is perfectly suited for their industrial use in cosmetic formulations, even many months after their preparation.

In the present text with the expression "anionic acrylic monomers" we mean both acrylic monomers containing a strongly acidic functional group, at least some of which being in neutral salt form, and acrylic monomers containing a carboxylic group.

It is a fundamental object of the present invention an inverse emulsion for the preparation of cosmetic formulations wherein the weight ratio between the aqueous phase and the oil phase is from 4:1 to 2:1 and containing from 20 to 70% by weight of an anionic acrylic polymer obtained by inverse emulsion polymerisation of one or more anionic acrylic monomers, at least one of which containing a strongly acidic functional group, dissolved in the aqueous phase, and at least a hydrophobic acrylic monomer dissolved in the oil phase before the mixing of the two phases, the percentage of the hydrophobic acrylic monomers on the total weight of the anionic acrylic monomers being of 0.1% to 5% by weight, preferably of 0.5 to 1.5% by weight.

It is a further object of the present invention a procedure for the preparation of an inverse emulsion for cosmetic formulations characterised by:
a. adding to a mixture of water and one or more anionic acrylic monomer, at least one of which containing a strongly acidic functional group, an aqueous solution of an alkali to regulate the pH between 4 and 10, a cross-linking agent and an initiator of radical polymerisation, maintaining the temperature between 0° and 5°C;
b. preparing an oil phase containing from 0.1 to 10% by weight of at least one hydrophobic acrylic monomer and one or more water-in-oil emulsifiers;
c. introducing the mixture obtained in a. into the oil phase prepared in b. and emulsifying the two phases by vigorous stirring;
d. initiating the polymerisation and completing it, maintaining the temperature between 55° and 95°C, under vigorous stirring;
e. cooling the reaction mixture to 35-45°C and adding an oil-in-water emulsifier.

The anionic acrylic monomer containing a strongly acidic functional group is selected among the monomer of this kind that are normally employed for the preparation of polymeric synthetic thickeners for the cosmetic use, such as 2-acrylamido-2-methylpropanesulfonic acid and its salts.

In the present text with the expression "hydrophobic acrylic monomer" we mean an acrylic monomer which is insoluble in water.

For the realisation of the present invention the preferred hydrophobic acrylic monomers are esters of acrylic or methacrylic acid with C₄-C₂₀ linear or branched monofunctional alcohols; the more preferred hydrophobic acrylic monomers are stearyl methacrylate and n-butyl acrylate.

In the preferred form of realisation of the present invention the anionic acrylic monomers dissolved in the aqueous phase are a mixture of at least one monomer containing a strongly acidic functional group (AF) and one or more monomers containing a carboxylic group (AC), the weight ratio between AF and AC being comprised from 4:1 and 1:1, more preferably from 2.5:1 and 1.5:1.

Preferably the anionic acrylic monomers containing a carboxylic group are chosen between acrylic acid and methacrylic acid.

In the procedure of the invention, normally, the alkali used is NaOH.

According to a preferred aspect of the invention the anionic acrylic polymer obtained by inverse emulsion polymerisation is cross-linked with from 0.01% to 1% by weight on the total weight of the monomers of a compound containing two or more ethylenic groups, more preferably with methylene-bis-acrylamide,

Among the initiators of radical polymerisation utilisable for the realisation of the present invention are ammonium, potassium or sodium persulfate, and watersoluble organic peroxides, by way of example hydrogen peroxide and peracetic acid.

For the realisation of the present invention it is also possible to use an initiator of radical polymerisation which is soluble in the oil phase containing the hydrophobic acrylic monomer; examples of such initiators are lauroyl peroxide and benzoyl peroxide.

In the inverse emulsions of the invention the oil phase consists of mineral oils containing saturated hydrocarbons or by vegetable oils or by mixture thereof having boiling point from 150 to 300°C.

Preferably the organic phase is a C₁₃-C₁₆ iso-paraffin.

The water-in-oil and the oil-in-water emulsifiers are those normally used for this purpose.

We cite among the utilisable water-in-oil emulsifiers: sorbitan monolaurate, sorbitan monopalmitate, sorbitan monostearate, sorbitan monooleate; among the utilisable oil-in-water emulsifiers we cite the linear or branched ethoxylated alcohols.

To initiate the polymerisation of the acrylic monomers advantageously an aqueous solution of sodium metabisulfite is used.

The inverse emulsions of the invention may further additionally contain the common additives used in radical polymerisation, by way of example sequestering agents such as sodium diethylenetriaminepentaacetate .

As it was previously observed, the inverse emulsions of the present invention are stable and allow the obtainment of stable cosmetic formulations; without giving an exhaustive explanation of the phenomenon it is supposed that the presence of hydrophobic side chains in the polymeric structure enhances the compatibility of the thickener with all the other organic compounds.

Polymers incorporating hydrophobic side chains are part of the state of the art of other categories of products, such as polymeric surfactants, which are however used for their surface-active properties and do not possess thickening properties.

In the following examples the preparation of inverse emulsions according to the invention and of some cosmetic formulations containing them is reported.

The following examples illustrate the present invention.

### Example 1.

The following ingredients are loaded into a 1.5 l pirex reactor equipped with a steel anchor stirrer:
62.21 g deionised water,
573 g aqueous solution (50% by weight) of sodium 2-acrylamido-2-methylpropane sulfonate;
135 g acrylic acid;

After a cooling down period, necessary to reach a temperature close to 0°C, the following ingredient are slowly added while stirring:
112.38 g aqueous solution (50% by weight) of NaOH;
10 g aqueous solution (1% by weight) of methylene-bisacrylamide;
0.5 g aqueous solution (40% by weight) of sodium diethylenetriaminepentaacetate;
10.75 g aqueous solution (4% by weight) of ammonium persulfate.

In the meantime, the organic phase is prepared inside a 500 ml beaker adding under stirring:
20 g sorbitan monooleate;
4.2 g stearyl methacrylate;
214.8 g C₁₃-C₁₆ hydrocarbon isoparaffin.

The aqueous phase is slowly added into the organic phase and subsequently the mixture is efficiently stirred with a high shear dispersing machine (ultra-turrax IKA). The emulsion obtained is then reloaded in the reactor and the reaction is ready to be started (reaction phase). The first operation is to insufflate nitrogen directly in the bulk of the product for about 10 minutes. This is a key step, because it enables to lower and control the amount of oxygen dissolved in the emulsion and to adjust the induction times. The second phase takes place only after the emulsion temperature is warmed up to 20°C. After that, 21.5 g of a 1% by weight aqueous solution of sodium metabisulfite is quickly loaded drop-wise through an addition funnel. The third phase is the radical reaction. The reaction proceeds spontaneously raising gradually the temperature to about 60 °C in 50 minutes. The stirring is maintained very fast and cool water re-circulates inside the reactor jacket. After this period of time the emulsion is kept at 60°C for about one hour to complete the monomers conversion, consuming the residual monomers. Subsequently a cooling down period is required to reach a temperature of 35-40°C. The final step is the addition of 25 g of C₁₂-C₁₆ (8 moles) ethoxylated linear alcohol.

The mixture is rapidly stirred till homogeneity is reached; the final emulsion (Emulsion 1) is then unloaded and stored for at least 24 hours before the evaluation of its properties.

Property evaluation of Emulsion 1.

Samples of Emulsion 1 are stored at different temperatures.

The emulsion stability is evaluated at different temperatures by visually checking possible phase separation or settling on the bottom of the vessel using a glass stick. In the following table (Table 1) the test temperatures and minimal stability times of the emulsion are shown.

**Table 1**

| | | | |
|---|---|---|---|
| Temperature | -3°C | 20°C | 45°C |
| Stability (days) | >30 | >100 | >30 |

The thickening properties are instead evaluated as follows and are shown in Tables 2 and 3.

A 2% by weight aqueous solution of Emulsion 1 is prepared in deionised water with high stirring in a 1 litre beaker.

Subsequently the viscosity is measured at 20°C, at different pH values (see Table 2) and adding different concentration of electrolyte (NaCl, as shown in Table 3).

The pH was adjusted by additions of an aqueous solution (50%) of citric acid.

**Table 2. Brookfield Viscosity in mPa.s (spindle 6, after 24 h)**

| 5 rpm | 10 rpm | pH |
|---|---|---|
| 53800 | 31000 | 7.5 |
| 51600 | 30600 | 7.0 |
| 20000 | 12900 | 6.87 |
| 3000 | 2200 | 6.47 |
| 1000 | 600 | 4.3 |

| | | |
|---|---|---|
| rpm= rounds per minute | | |

**Table 3. Brookfield Viscosity in mPa.s (spindle 6, after 24 h, pH =7.5)**

| | 0% NaCl | 0.1% NaCl | 0.2% NaCl | 0.3% NaCl | 0.4% NaCl |
|---|---|---|---|---|---|
| 5 rpm | 53800 | 45200 | 34800 | 31000 | 20000 |
| 10 rpm | 31000 | 23000 | 19600 | 18400 | 12400 |

| | | | | | |
|---|---|---|---|---|---|
| rpm= rounds per minute | | | | | |

### Example 2

An inverse emulsion is prepared as described in Example 1, substituting stearyl methacrylate in the oil phase with 5 g of butyl acrylate thus obtaining Emulsion 2.

Property evaluation of Emulsion 2.

Samples of Emulsion 2 are stored at different temperatures.

The emulsion stability is evaluated at different temperatures by visually checking possible phase separation or settling on the bottom of the vessel using a glass stick.

In the following table (Table 4) the test temperatures and minimal stability times of the emulsion are shown.

**Table 4**

| | | | |
|---|---|---|---|
| Temperature | -3°C | 20°C | 45°C |
| Stability (days) | >40 | >200 | >40 |

The thickening properties are evaluated as described for Emulsion 1 and are shown in Tables 5 and 6.

**Table 5 . Brookfield Viscosity in mPa.s (spindle 6, after 24 h)**

| 5 rpm | 10 rpm | pH |
|---|---|---|
| 65600 | 38000 | 7.37 |
| 13800 | 8600 | 6.84 |
| 6800 | 4300 | 6.12 |
| 600 | 500 | 4.51 |

| | | |
|---|---|---|
| rpm= rounds per minute | | |

**Table 6. Brookfield Viscosity in mPa.s (spindle 6, after 24 h, pH =7.5)**

| | 0%NaCl | 0.1% NaCl | 0.2% NaCl | 0.3% NaCl | 0.4% NaCl |
|---|---|---|---|---|---|
| 5 rpm | 65600 | 52000 | 35000 | 27000 | 20200 |
| 10 rpm | 38000 | 30500 | 20700 | 16500 | 12200 |

| | | | | | |
|---|---|---|---|---|---|
| rpm= rounds per minute | | | | | |

### Example 3.

A body cream is prepared using Emulsion 1; all the ingredients are listed in Table 7 and the procedure is described hereinafter.

Phase A is prepared by homogenising all the ingredients at room temperature and then heating the mixture to 70 °C.

Phase B is prepared heating all the ingredients to 70-75°C; Phase A is added to Phase B stirring vigorously. The mixture is cooled down to 40°C and Phase C and

Phase D are added, stirring till homogeneity is reached.

Properties of the cream obtained:
Viscosity = 29000 mPa.s (5 rpm, spindle 4); 43000 mPa.s (2.5 rpm spindle 4); pH=7.5

**Table 7. Body cream.**

| Ingredients | % |
|---|---|
| Phase A | |
| Aqua | to 100 |
| Glycerin | 3 |
| EDTA | 0.1 |
| Emulsion 1 | 0.4 |

| Phase B | |
|---|---|
| Polydecene | 15 |
| Prunus Amygdalus dulcis | 5 |
| Caprylic/capric triglyceride | 4 |
| Steareth-2 | 2 |
| Steareth-21 | 3 |

| Phase C | |
|---|---|
| Preservative | 1 |

| Phase D | |
|---|---|
| Parfum | 0.1 |

### Example 4

A foundation is prepared using Emulsion 2; all the ingredients are listed in Table 8 and the procedure is described hereinafter.

All the ingredients of Phase B are mixed and stirred till homogeneity is reached. Phase A is prepared by mixing all its ingredients and heating to 70°C; then Phase

A is added to Phase B. The mixture of the two phases is homogenised and then cooled down to 40°C.

Phase C and D are added while stirring.

Properties of the foundation:
Viscosity = 28000 mPa.s (5 rpm, spindle 4); 46000 mPa.s (2.5 rpm, spindle 4); pH=7.0.
Stability: No separation after 60 minutes of centrifugation at 6000 rpm.

**Table 8. Foundation.**

| Ingredients | % |
|---|---|
| Phase A | |
| Aqua | to 100 |
| Glycerin | 3 |
| EDTA | 0.1 |
| Emulsion 2 | 0.4 |

| Phase B | |
|---|---|
| Polydecene | 15 |
| Prunus Amygdalus dulcis | 5 |
| Caprylic/capric triglyceride | 4 |
| Steareth-2 | 2 |
| Steareth-21 | 3 |
| Unipure Brown LC889 * | 8 |
| Unipure Yellow LC182 * | 1 |
| Unipure White LC 981 * | 1 |

| Phase C | |
|---|---|
| Preservative | 1 |

| Phase D | |
|---|---|
| Parfum | 0.1 |

| | |
|---|---|
| *pigments sold by LCW (France) | |

### Example 5

A moisturising cream is prepared using Emulsion 1; all the ingredients are listed in Table 9 and the procedure is described hereinafter.

All the ingredients of Phase A are mixed and stirred till homogeneity is reached. Phase B is added and the mixture is heated to 75°C. All the ingredients of Phase C are mixed under vigorous stirring at 75°C, then Phase A and Phase B are added to Phase C till homogeneity is reached. The mixture of the phases is then cooled down to 40°C and the ingredients of Phase D, E, F and G are added while stirring.

**Table 9. Moisturising cream.**

| Ingredients | % |
|---|---|
| Phase A | |
| Aqua | to 100 |
| Glycerin | 8 |
| EDTA | 0.1 |
| Panthenol | 0.3 |
| Micronised TiO₂ | 2 |
| Emulsion 1 | 2 |

| Phase B | |
|---|---|
| Nylon-12 | 2 |

| Phase C | |
|---|---|
| Polydecene | 15 |
| Cetyl alcohol | 2 |
| Bis-hydroxyethyl bis cetyl malonamide | 0.1 |

| Phase D | |
|---|---|
| Preservative | 1 |
| Tocopheryl acetate | 0.5 |

| Phase E | |
|---|---|
| Citrus aurantium dulcis | 5 |
| Yeast | 2 |

| Phase F | |
|---|---|
| Beta-glucan | 1 |

| Phase G | |
|---|---|
| Parfum | 0.1 |

Properties of the cream:
Viscosity = 36000 mPa.s (5 rpm, spindle 5); 67600 mPa.s (2.5 rpm, spindle 5); pH=6.75.
Stability.
   No separation after 60 minutes of centrifugation at 6000 rpm.

### Example 6.

A body cream is prepared using Emulsion 2; all the ingredients are listed in Table 10 and the procedure is described hereinafter.

**Table 10. Body cream.**

| Ingredients | % |
|---|---|
| Phase A | |
| Aqua | to 100 |
| Glycerin | 4 |
| Emulsion 2 | 0.6 |

| Phase B | |
|---|---|
| Mineral oil | 8 |
| Isopropyl palmitate | 5 |
| Octyl stearate | 4 |
| Polyglyceryl 2-stearate | 2 |

| Phase C | |
|---|---|
| Coco sodium glutamate | 2 |

| Phase D | |
|---|---|
| Preservative | 1 |
| Parfum | 0.1 |

All the ingredients of Phase B are heated to 70°C and are stirred till homogeneity is reached.

Phase A is prepared by mixing and heating all the ingredients at 70°C and then it is added to Phase B. The mixture is homogenised and cooled down to 40°C. Phase C and D are added under stirring.

Properties of the cream obtained:
Viscosity = 25000 mPa.s (5 rpm, spindle 4); 36000 mPa.s (2.5 rpm spindle 4) pH=7.0
Stability.
   No separation after 60 minutes of centrifugation at 6000 rpm.

### Example 7.

A skin cleansing lotion is prepared using Emulsion 2; all the ingredients are listed in Table 12 and the procedure is described hereinafter.

All the ingredients of Phase B are heated to 70°C and are stirred till homogeneity is reached.

Phase A is prepared by mixing and heating all the ingredients at 70°C and then it is added to Phase B. The mixture is homogenised and cooled down to 40°C. Phase C and D are added under stirring.

Properties of the lotion obtained:
Viscosity = 10000 mPa.s (5 rpm, spindle 4); 16000 mPa.s (2.5 rpm spindle 4); pH=6.5
Stability.
No separation after 60 minutes of centrifugation at 6000 rpm.

**Table 12. Skin cleansing lotion.**

| Ingredients | % |
|---|---|
| Phase A | |
| Aqua | to 100 |
| Emulsion 2 | 0.7 |

| Phase B | |
|---|---|
| Almond oil | 1 |
| Isopropyl palmitate | 4 |
| Wheat germ oil | 1 |
| Cetearyl Isononanoate | 8 |

| Phase C | |
|---|---|
| Polyglyceryl-2-polyethyleneglycol-10-laurate | 2 |

| Phase D | |
|---|---|
| Preservative | 1 |
| Parfum | 0.3 |

### Example 8.

An after-shave balm is prepared using Emulsion 1; all the ingredients are listed in Table 13 and the procedure is described hereinafter.

All the ingredients of Phase A are mixed and stirred till homogeneity is reached. Phase B and C are added under stirring.

**Table 13. After-shave balm.**

| Ingredients | % |
|---|---|
| Phase A | |
| Aqua | to 100 |
| Emulsion 1 | 0.7 |

| Phase B | |
|---|---|
| Ethanol 95% | 10 |
| Wheat germ oil | 2 |

| Phase C | |
|---|---|
| Preservative | 1 |
| Parfum | 0.5 |

Properties of the balm obtained:
Viscosity = 2000 mPa.s (5 rpm, spindle 4); 4000 mPa.s (2.5 rpm spindle 4) pH=6.5
Stability:
   No separation after 60 minutes of centrifugation at 6000 rpm.

### Example 9.

A skin protectant is prepared using Emulsion 1; all the ingredients are listed in Table 14 and the procedure is described hereinafter.

All the ingredients of Phase A are heated to 40°C and stirred till homogeneity is reached. Phase B is added under stirring. Phase C is added and the mixture is homogenised.

Properties of the cream obtained:
Viscosity = 10000 mPa.s (5 rpm, spindle 4); 14000 mPa.s (2.5 rpm spindle 4); pH=7.0
Stability.
No separation after 60 minutes of centrifugation at 6000 rpm.

**Table 14. Skin protectant.**

| Ingredients | % |
|---|---|
| Phase A | |
| Aqua | to 100 |
| Emulsion 1 | 0.7 |
| Glycerin | 3 |
| EDTA | 0.05 |
| Lysine | 0.025 |

| Phase B | |
|---|---|
| Clyclomethicone | 10 |
| Wheat germ oil | 1 |

| Phase C | |
|---|---|
| Preservative | 1 |
| Parfum | 0.5 |

### Example 10.

A Massage Gel is prepared using Emulsion 1; all the ingredients are listed in Table 15 and the procedure is described hereinafter.

**Table 15. Massage Gel**

| Ingredients | % |
|---|---|
| Phase A | |
| Aqua | to 100 |
| Emulsion 1 | 1.2 |
| Dye | 0.001 |

| Phase B | |
|---|---|
| Ethanol 95% | 10 |
| Menthol | 0.10 |

| Phase C | |
|---|---|
| Preservative | 1 |
| Parfum | 0.5 |

All the ingredients of Phase A are mixed and stirred till homogeneity is reached.

All the ingredients of Phase B and C are added under stirring.

Properties of the gel obtained:
Viscosity = 40000 mPa.s (5 rpm, spindle 4); 80000 mPa.s (2.5 rpm spindle 4) pH=6.5
Stability.
   No separation after 60 minutes of centrifugation at 6000 rpm.

### Example 11.

A skin protectant is prepared using Emulsion 1; all the ingredients are listed in Table 16 and the procedure is described hereinafter.

**Table 16. Skin protectant**

| Ingredients | % |
|---|---|
| Phase A | |
| Aqua | to 100 |
| Emulsion 1 | 0.7 |
| Glycerin | 3 |
| EDTA | 0.05 |
| Lysine | 0.025 |

| Phase B | |
|---|---|
| Cyclomethicone | 10 |
| Wheat germ oil | 1 |

| Phase C | |
|---|---|
| Preservative | 1 |
| Parfum | 0.5 |

All the ingredients of Phase A are mixed and stirred till homogeneity is reached.

All the ingredients of Phase B and C are added under stirring.

Properties of the cream obtained:
Viscosity = 10000 mPa.s (5 rpm, spindle 4); 14000 mPa.s (2.5 rpm spindle 4) pH=7.0
Stability.
   No separation after 60 minutes of centrifugation at 6000 rpm.

## Claims

1. Stable inverse emulsion wherein the weight ratio between the aqueous phase and the oil phase is from 4:1 to 2:1 and containing from 20 to 70% by weight of an anionic acrylic polymer obtainable by inverse emulsion polymerisation of one or more anionic acrylic monomers, at least one of which containing a strongly acidic functional group, dissolved in the aqueous phase, and at least a hydrophobic acrylic monomer dissolved in the oil phase before the mixing of the two phases, the percentage of the hydrophobic acrylic monomers on the total weight of the anionic acrylic monomers being from 0.1% to 5% by weight.

2. Stable inverse emulsion according to claim 1., wherein the percentage of the hydrophobic acrylic monomers on the total weight of the anionic acrylic monomers is from 0.5 to 1.5% by weight.

3. Stable inverse emulsion according to claim 1. or 2., wherein the anionic acrylic monomer is 2-acrylamido-2-methylpropanesulfonic acid and/or its sodium salt.

4. Stable inverse emulsion according to claim 3., wherein the hydrophobic acrylic monomer are esters of acrylic or methacrylic acid with C₄-C₂₀ linear or branched monofunctional alcohols.

5. Stable inverse emulsion according to claim 4., wherein the hydrophobic acrylic monomer is stearyl methacrylate or n-butyl methacrylate.

6. Procedure for the preparation of an inverse emulsion **characterised by**:
a. adding to a mixture of water and one or more anionic acrylic monomer, at least one of which containing a strongly acidic functional group, an aqueous solution of an alkali to regulate the pH between 4 and 10, a cross-linking agent and an initiator of radical polymerisation, maintaining the temperature between 0° and 5°C;
b. preparing an oil phase containing from 0.1 to 10% by weight of at least one hydrophobic acrylic monomer and one or more water-in-oil emulsifiers;
c. introducing the mixture obtained in a. into the oil phase prepared in b. and emulsifying the two phases by vigorous stirring;
d. initiating the polymerisation and completing it maintaining the temperature between 55° and 95°C undervigorous stirring;
e. cooling the reaction mixture to 35-45°C and adding an oil-in-water emulsifier.

7. Procedure for the preparation of an inverse emulsion according to claim 6., wherein the anionic acrylic monomer containing a strongly acidic functional group is 2-acrylamido-2-methylpropanesulfonic acid and/or its sodium salt.

8. Procedure for the preparation of an inverse emulsion according to claim 7., wherein the hydrophobic acrylic monomers are esters of acrylic or methacrylic acid with C₄-C₂₀ linear or branched monofunctional alcohols.

9. Procedure for the preparation of an inverse emulsion according to claim 8., wherein the hydrophobic acrylic monomers are stearyl methacrylate or n-butyl acrylate.

10. Procedure for the preparation of an inverse emulsion according to claim 9., wherein the anionic acrylic monomers dissolved in the aqueous phase are a mixture of at least one monomer containing a strongly acidic functional group (AF) and one or more monomers containing a carboxylic group (AC), the weight ratio between AF and AC being comprised from 4:1 and 1:1.

11. Procedure for the preparation of an inverse emulsion according to claim 10., wherein the anionic acrylic monomers containing a carboxylic group are chosen among acrylic acid and methacrylic acid.

12. Procedure for the preparation of an inverse emulsion according to any of the claims from 6. to 11., wherein the anionic acrylic polymer obtained by inverse emulsion polymerisation is cross-linked with from 0.01% to 1 % by weight on the total weight of the monomers of a compound containing two or more ethylenic groups.

13. Procedure for the preparation of an inverse emulsion according to claim 12., wherein the compound containing two or more ethylenic groups is methylene-bis-acrylamide.

## Patentansprüche

1. Stabile Umkehremulsion, worin das Gewichtsverhältnis zwischen wässriger Phase und öliger Phase zwischen 4:1 und 2:1 liegt und die zwischen 20 und 70% Gewichtsanteile eines anionischen Acrylpolymers enthält, den man durch Umkehremulsionspolymerisation eines oder mehrerer anionischen/r Acrylmonomers/e enthält, von denen mindestens einer eine stark saure und in der wässrigen Phase gelöste Funktionsgruppe und mindestens einen vor dem Mischen der beiden Phasen in der öligen Phase gelösten hydrophoben Acrylmonomer enthält, wobei der Prozentsatz der hydrophoben Acrylmonomere am Gesamtgewicht der anionischen Acrylmonomere einen Gewichtsanteil zwischen 0.1 % und 5% ausmacht.

2. Stabile Umkehremulsion entsprechend Patentanspruch 1., worin der Prozentsatz der hydrophoben Acrylmonomere am Gesamtgewicht der anionischen Acrylmonomere einen Gewichtsanteil zwischen 0.5 und 1.5% ausmacht.

3. Stabile Umkehremulsion entsprechend Patentanspruch 1. oder 2., worin der anionische Acrylmonomer eine 2-Acrylamid-2-Methylpropansulfonsäure und/oder dessen Natriumsalz ist.

4. Stabile Umkehremulsion entsprechend Patentanspruch 3., worin die hydrophoben Acrylmonomere Ester der Acryl- oder Methacrylsäure mit C₄-C₂₀ linearen oder verzweigten monofunktionalen Alkoholen sind.

5. Stabile Umkehremulsion entsprechend Patentanspruch 4., worin der hydrophobe Acrylmonomer ein Stearylmethacrylat oder ein n-Butylmethacrylat ist.

6. Vorbereitungsprozedur einer Umkehremulsion, **dadurch charakterisiert, dass:**
a. man in eine Mischung aus Wasser und einem oder mehreren anionischen Acrylmonomeren, von denen mindestens einer eine stark saure Funktionsgruppe enthält, eine wässrige Alkalilösung zur Regelung des pH zwischen 4 und 10, ein Vernetzungsmittel und einen Radikalpolymerisations-Initiator beimengt, und die Temperatur zwischen 0° und 5°C hält;
b. man eine ölige Phase vorbereitet, die zwischen 0.1 und 10% Gewichtsanteile an mindestens einem hydrophoben Acrylmonomer und einen oder mehrere Wasser-in-Öl-Emulgator/en enthält;
c. man die in a. erhaltene Mischung in die in b. vorbereitete Ölphase mengt und die beiden Phasen durch energisches Rühren emulgiert;
d. man die Polymerisation beginnt und durch Halten der Temperatur auf 55° bis 95°C unter energischem Rühren vollendet;
e. man die Reaktionsmischung auf 35-45°C abkühlt und einen Öl-in-Wasser-Emulgator beimengt.

7. Vorbereitungsprozedur einer Umkehremulsion entsprechend Patentanspruch 6., worin der eine stark saure Funktionsgruppe enthaltende anionische Acrylmonomer eine 2-Acrylamid-2-Methylpropansulfonsäure und/oder dessen Natriumsalz ist.

8. Vorbereitungsprozedur einer Umkehremulsion entsprechend Patentanspruch 7., worin die hydrophoben Acrylmonomere Ester der Acryl- oder Methacrylsäure mit C₄-C₂₀ linearen oder verzweigten monofunktionalen Alkoholen sind.

9. Vorbereitungsprozedur einer Umkehremulsion entsprechend Patentanspruch 8., worin die hydrophoben Acrylmonomere Stearylmethacrylate oder n-Butylacrylate sind.

10. Vorbereitungsprozedur einer Umkehremulsion entsprechend Patentanspruch 9., worin die in wässriger Phase gelösten anionischen Acrylmonomere eine Mischung aus mindestens einem Monomer sind, der eine stark saure Funktionsgruppe (AF) und eine oder mehrere Carboxylgruppen (AC) enthält, wobei das Gewichtsverhältnis zwischen AF und AC zwischen 4:1 und 1:1 liegt.

11. Vorbereitungsprozedur einer Umkehremulsion entsprechend Patentanspruch 10., worin die eine Carboxylgruppe enthaltenden anionischen Acrylmonomere aus Acrylsäure oder Methacrylsäure gewählt werden.

12. Vorbereitungsprozedur einer Umkehremulsion entsprechend jedem der Patentansprüche von 6. bis 11., worin der durch Umkehremulsionspolymerisation erhaltene anionische Acrylpolymer mit einem Gewichtsanteil von 0.01% bis 1 % des Gesamtgewichts der Monomere einer Verbindung vernetzt wird, die zwei oder mehrere Ethylengruppen enthält.

13. Vorbereitungsprozedur einer Umkehremulsion entsprechend Patentanspruch 12., worin die zwei oder mehr Ethylengruppen enthaltende Verbindung ein Methylen-bis-Acrylamid ist.

## Revendications

1. Émulsion inverse stable dans laquelle le rapport pondéral entre la phase aqueuse et la phase huileuse va de 4/1 à 2/1, et contenant de 20 à 70 % en poids d'un polymère acrylique anionique qu'il est possible d'obtenir par polymérisation en émulsion inverse d'un ou plusieurs monomères acryliques anioniques, dont l'un au moins contient un groupe fonctionnel fortement acide, dissous dans la phase aqueuse, et au moins un monomère acrylique hydrophobe dissous dans la phase huileuse avant mélange des deux phases, le pourcentage des monomères acryliques hydrophobes par rapport au poids total de monomères acryliques anioniques allant de 0,1 % à 5 % en poids.

2. Émulsion inverse stable selon la revendication 1., dans laquelle le pourcentage de monomères acryliques hydrophobes par rapport au poids total de monomères acryliques anioniques va de 0,5 à 1,5 % en poids.

3. Émulsion inverse stable selon la revendication 1. ou 2., dans laquelle le monomère acrylique anionique est l'acide 2-acrylamido-2-méthylpropanesulfonique et/ou son sel de sodium.

4. Émulsion inverse stable selon la revendication 3., dans laquelle les monomères acryliques hydrophobes sont des esters de l'acide acrylique ou méthacrylique avec des alcools monofonctionnels linéaires ou ramifiés en C₄ à C₂₀.

5. Émulsion inverse stable selon la revendication 4., dans laquelle le monomère acrylique hydrophobe est le méthacrylate de stéaryle ou le méthacrylate de n-butyle.

6. Mode opératoire de préparation d'une émulsion inverse **caractérisé par** :
a. l'ajout, à un mélange d'eau et d'un ou plusieurs monomères acryliques anioniques, dont l'un au moins contient un groupe fonctionnel fortement acide, d'une solution aqueuse d'un alcali pour réguler le pH entre 4 et 10, d'un agent de réticulation et d'un initiateur de polymérisation radicalaire, en maintenant la température entre 0 ° et 5 °C ;
b. la préparation d'une phase huileuse contenant de 0,1 à 10 % en poids d'au moins un monomère acrylique hydrophobe et un ou plusieurs émulsifiants eau-dans-huile ;
c. l'introduction du mélange obtenu en a. dans la phase huileuse préparée en b. et l'émulsification des deux phases au moyen d'une agitation vigoureuse ;
d. l'initiation de la polymérisation et son déroulement intégral en maintenant la température entre 55 ° et 95 °C, sous agitation vigoureuse ;
e. le refroidissement du mélange réactionnel jusqu'à 35 à 45 °C et l'ajout d'un émulsifiant huile-dans-eau.

7. Mode opératoire de préparation d'une émulsion inverse selon la revendication 6., où le monomère acrylique anionique contenant un groupe fonctionnel fortement acide est l'acide 2-acrylamido-2-méthylpropanesulfonique et/ou son sel de sodium.

8. Mode opératoire de préparation d'une émulsion inverse selon la revendication 7., où les monomères acryliques hydrophobes sont des esters de l'acide acrylique ou méthacrylique avec des alcools monofonctionnels linéaires ou ramifiés en C₄ à C₂₀.

9. Mode opératoire de préparation d'une émulsion inverse selon la revendication 8., où les monomères acryliques hydrophobes sont le méthacrylate de stéaryle ou l'acrylate de n-butyle.

10. Mode opératoire de préparation d'une émulsion inverse selon la revendication 9., où les monomères acryliques anioniques dissous dans la phase aqueuse sont un mélange d'au moins un monomère contenant un groupe fonctionnel fortement acide (AF) et un ou plusieurs monomères contenant un groupe carboxylique (AC), le rapport pondéral entre AF et AC étant compris entre 4/1 et 1/1.

11. Mode opératoire de préparation d'une émulsion inverse selon la revendication 10., où les monomères acryliques anioniques contenant un groupe carboxylique sont choisis parmi l'acide acrylique et l'acide méthacrylique.

12. Mode opératoire de préparation d'une émulsion inverse selon l'une quelconque des revendications 6. à 11., où le polymère acrylique anionique obtenu par polymérisation en émulsion inverse est réticulé avec de 0,01 % à 1 % en poids par rapport au poids total des monomères d'un composé contenant deux groupes éthyléniques ou plus.

13. Mode opératoire de préparation d'une émulsion inverse selon la revendication 12., où le composé contenant deux groupes éthyléniques ou plus est le méthylène-bis-acrylamide.
